(19) Europäisches Patentamt
European Patent Office
Office européen des brevets

(11) **EP 3 945 087 B1**

(12) **EUROPÄISCHE PATENTSCHRIFT**

(45) Veröffentlichungstag und Bekanntmachung des Hinweises auf die Patenterteilung:
**13.09.2023   Patentblatt 2023/37**

(21) Anmeldenummer: **20188048.1**

(22) Anmeldetag: **28.07.2020**

(51) Internationale Patentklassifikation (IPC):
**C07C 67/303** (2006.01)   **C07C 69/75** (2006.01)

(52) Gemeinsame Patentklassifikation (CPC):
(C-Sets verfügbar)
**C07C 69/75; C07C 67/303;** Y02P 20/582   (Forts.)

(54) **VERFAHREN ZUR HERSTELLUNG VON 1,4-CYCLOHEXANDICARBONSÄUREDIALKYLESTERN**

METHOD FOR THE PREPARATION OF 1,4-CYCLOHEXANE DICARBOXYLIC ACID DIALKYL ESTERS

PROCÉDÉ DE FABRICATION D'ESTERS DIALKYLIQUES DE L'ACIDE 1,4-CYCLOHEXANEDICARBOXYLIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**02.02.2022   Patentblatt 2022/05**

(73) Patentinhaber: **Evonik Operations GmbH**
**45128 Essen (DE)**

(72) Erfinder:
- **KRAFT, Johannes**
  **45770 Marl (DE)**
- **ALTMANN, Lena**
  **46282 Dorsten (DE)**
- **ANTON, Johan**
  **46284 Dorsten (DE)**
- **GRASS, Michael**
  **45721 Haltem Am See (DE)**
- **SCHNEIDER, Thomas**
  **46514 Schermbeck (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A1- 3 085 686**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C07C 67/303, C07C 69/75**

**Beschreibung**

Die vorliegende Erfindung betrifft ein Verfahren zur Herstellung von 1,4-Cyclohexandicarbonsäuredialkylestern durch Kernhydrieren des entsprechenden Dialkylterephthalats, welches eine CO-Zahl von weniger als 0,3 mg KOH/g aufweist. Auch offenbart ist die Verwendung der so hergestellten 1,4-Cyclohexandicarbonsäuredialkylester als Weichmacher oder als Teil einer Weichmacherzusammensetzung für Kunststoffe, insbesondere PVC zum Gegenstand. EP 3085686 offenbart die Hydrierung von Terephthalsäureestern zu 1,4-Cyclohexan-Dicarbonsäureestern.

Weichmacher werden in vielen technischen Bereich eingesetzt, um Kunststoffe wie Polyvinylchlorid (PVC) weicher und biegsamer zu machen. Seit vielen Jahren sind Phthalate, also die Diester der (ortho-) Phthalsäure, die dominierende Weichmacherklasse. In den letzten Jahren haben aber auch die Alkylester von Cyclohexandicarbonsäuren an Bedeutung gewonnen, nicht zuletzt wegen der Diskussion um eventuelle Gesundheitsbedenken der Phthalat-basierten Weichmacher. Dabei spielen vor allem die 1,2-Cyclohexandicarbonsäuredialkylester und neuerdings auch die 1,4-Cyclohexandicarbonsäuredialkylester eine Rolle.

1,2-, 1,3- und 1,4-Cyclohexandialkyldicarbonsäureester können durch die Hydrierung des aromatischen Rings der entsprechenden Phthalate, Isophthalate oder Terephthalate (nachfolgend: Kernhydrierung) hergestellt werden. Teilweise werden solche Kernhydrierungen bereits großtechnisch durchgeführt, beispielsweise für die Herstellung von DINCH, dem Diisononylester der 1,2-Cyclohexandicarbonsäure.

Die Reaktionsgeschwindigkeit der Kernhydrierung spielt dabei eine wichtige Rolle für die Wirtschaftlichkeit des Verfahrens, da sowohl die Investitions- als auch die Betriebskosten hiervon beeinflusst werden.

Die Aufgabe der vorliegenden Erfindung bestand deshalb darin, ein Verfahren zur Herstellung von 1,4-Cyclohexandialkyldicarbonsäureestern bereitzustellen, mit dem die Hydriergeschwindigkeit auf höherem Niveau gehalten und somit die Produktivität des Hydrierprozesses gesteigert werden kann.

Überraschend wurde nun gefunden, dass Dialkylterephthalate, bei denen die Alkylgruppen jeweils mindestens 2 Kohlenstoffatome, vorzugsweise mindestens 4 Kohlenstoffatome aufweisen, sich leichter und schneller hydrieren lassen, wenn deren Carbonylzahl (CO-Zahl) einen bestimmten Wert nicht überschreitet. Werden also entsprechende Dialkylterephthalate bei der Kernhydrierung eingesetzt, steigt die Produktivität und damit auch die Wirtschaftlichkeit des Verfahrens.

Das erfindungsgemäße Verfahren ist demgemäß ein Verfahren zur Herstellung von 1,4-Cyclohexandicarbonsäuredialkylestern, bei denen die beiden Alkylgruppen jeweils mindestens 2 Kohlenstoffatome, vorzugsweise mindestens 4 Kohlenstoffatome aufweisen, wobei das Verfahren mindestens das Kernhydrieren eines Dialkylterephthalats, bei dem die beiden Alkylgruppen jeweils mindestens 2 Kohlenstoffatome, vorzugsweise mindestens 4 Kohlenstoffatome aufweisen, mit einem Wasserstoff-haltigen Gas zum entsprechenden 1,4-Cyclohexandicarbonsäuredialkylester umfasst, wobei das bei der Kernhydrierung eingesetzte Dialkylterephthalat eine CO-Zahl von weniger als 0,3 mg KOH/g, vorzugsweise weniger als 0,2 mg KOH/g, besonders bevorzugt weniger als 0,1 mg KOH/g aufweist.

Die CO-Zahl ist definiert als die Menge KOH in Milligramm, die der zur Oximierung von 1 g Substanz notwendigen Menge Hydroxylamin äquivalent sind. Die CO-Zahl wird darüber bestimmt, dass die in carbonylfreiem Alkohol gelöste Substanz mit einem Überschuss an Hydroxylamin zum entsprechenden Oxim umgesetzt und das unverbrauchte Hydroxylamin mit Salzsäure zurücktitriert wird.

Zur Bestimmung der CO-Zahl muss zunächst der Äquivalenzpunkt mit einer Kalibrierlösung bestimmt werden. Dazu werden Kalibrierlösungen mit unterschiedlichen Mengen an Cyclohexanon in einem geeigneten Lösemittel, z. B. carbonylfreiem Methanol hergestellt. Die theoretische CO-Zahl ergibt sich aus der nachfolgenden Formel:

$$\text{CO-Zahl (theoretisch)} = \frac{Molmasse\ KOH \cdot Reinheit\ Cyclohexanon}{Molmasse\ Cyclohexanon}$$

Die hergestellten Kalibrierlösungen werden jeweils mit 0,1 mol/l Salzsäure titriert. Der gemessene pH-Wert wird dann als Funktion des jeweiligen Salzsäureverbrauchs aufgetragen und der Äquivalenzpunkt bestimmt. Damit ist das System kalibriert.

Die Bestimmung der CO-Zahl einer unbekannten Probe kann dann wie folgt bestimmt werden. Dazu wird zunächst eine geeignete Probenmenge in ein Reaktionsgefäß vorgelegt und in 50 ml eines geeigneten Lösemittels, z. B. carbonylfreiem Methanol gelöst. Mit dem eingesetzten Lösemittel, z. B. Methanol muss vorher eine Blindwertbestimmung ohne Probe wie nachfolgend beschrieben durchgeführt werden. Die Lösung der Probe in dem Lösemittel, z. B. Methanol wird mit Bromphenolblau versetzt und der pH-Wert - sofern erforderlich - durch Zugabe von Salzsäure oder Natronlauge so angepasst, dass eine grün-gelbe Färbung der Lösung (entspricht einem pH-Wert von etwa 3) vorhanden ist. Anschließend werden 20 ml Hydroxylaminlösung (c = 0,24 mol/l) zudosiert und die entstandene Lösung im Reaktionsgefäß 1 h unter Rückfluss gekocht.

Nach dem Abkühlen auf Raumtemperatur wird der Rückflusskühler mit 10 ml des Lösemittels, z. B. carbonylfreiem Methanol gespült und anschließend die Reaktionslösung mit 0,1 mol/l Salzsäure bis zum Äquivalenzpunkt titriert.

Die CO-Zahl kann dann anhand der nachfolgenden Formel berechnet werden:

$$\text{CO-Zahl (mg KOH/g)} = \frac{(V_B - V_H) \cdot F_{HCl} \cdot c_{HCl} \cdot M_{KOH}}{E_P}$$

wobei $V_B$ der Salzsäureverbrauch bei der Blindwertbestimmung in ml, $V_H$ der Salzsäureverbrauch bei der zu untersuchenden Probe in ml, $F_{HCl}$ der Titer von Salzsäure, $C_{HCl}$ die Konzentration der Salzsäure in mol/l, $M_{KOH}$ die Molmassen von KOH = 56,11 g/mol und $E_P$ die Probeneinwaage in g ist.

Sofern darauf geachtet wird, dass das bei der Kernhydrierung eingesetzte Dialkylterephthalat eine CO-Zahl von weniger als 0,3 aufweisen, lässt sich die Kernhydrierung schneller durchführen. Von den dadurch hergestellten 1,4-Cyclohexandicarbonsäuredialkylestern, bei denen die Alkylgruppen jeweils mindestens 2 Kohlenstoffatome, vorzugsweise mindestens 4 Kohlenstoffatome aufweisen, sind die 1,4-Cyclohexandicarbonsäuredialkylester bevorzugt, die 3 bis 10 Kohlenstoffatome, weiterhin bevorzugt 4 bis 10 Kohlenstoffatome, weiterhin bevorzugt 5 bis 9 Kohlenstoffatome, besonders bevorzugt 8 oder 9 Kohlenstoffatome und ganz besonders bevorzugt 9 Kohlenstoffatome aufweisen.

Die Kernhydrierung von Dialkylterephthalaten ist dem Fachmann grundsätzlich bekannt. Die Kernhydrierung wird mit einem Wasserstoff-haltigen Gas durchgeführt. Als Wasserstoff-haltiges Gas können grundsätzlich beliebige wasserstoffhaltige Gasgemische, die keine schädlichen Mengen an Katalysatorgiften, wie beispielsweise Kohlenmonoxid oder Schwefelwasserstoff enthalten, eingesetzt werden. Es können auch Gasgemische mit Inertgasen eingesetzt werden. Als Wasserstoff-haltiges Gas wird bevorzugt Wasserstoff in einer Reinheit ≥ 95 %, insbesondere ≥ 98 % eingesetzt. Inertgasanteile können beispielsweise Stickstoff oder Methan sein. Das Wasserstoffhaltige Gas wird vorzugsweise so eingesetzt, dass der Wasserstoff im Überschuss, insbesondere in einem Überschuss von bis zu 200 %, bevorzugt in einem Überschuss von 5 bis 100 % und besonders bevorzugt in einem Überschuss von 10 bis 50 % bezogen auf die stöchiometrische Menge, die zur Erzielung des gewünschten Umsatzes benötigt wird, vorliegt.

Bei der erfindungsgemäßen Kernhydrierung werden zudem heterogene Hydrierkatalysatoren eingesetzt, die vorzugsweise mindestens ein Übergangsmetall, besonders bevorzugt ein Metall der Gruppe 8 des Periodensystems der Elemente enthalten. Vorzugsweise werden als Übergangsmetall Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird. Neben den bereits genannten Metallen kann zusätzlich mindestens ein Metall der Gruppe 7 und 11 des Periodensystems der Elemente in den Katalysatoren enthalten sein. Bevorzugt werden Rhenium und/oder Kupfer eingesetzt.

Der Gehalt an Übergangsmetall im Hydrierkatalysator liegt vorzugsweise im Bereich von 0,1 bis 10 Gew.-%, insbesondere im Bereich von 0,5 bis 5 Gew.-%, ganz besonders im Bereich von 0,5 bis 2 Gew.-%.

Bevorzugt sind die eingesetzten heterogen Hydrierkatalysatoren Trägerkatalysatoren, d. h. sie umfassen ein Trägermaterial. Als Trägermaterial können Aktivkohle, Siliciumcarbid, Aluminiumoxid, Siliciumdioxid, Alumosilikat, Titandioxid, Zirkoniumdioxid, Magnesiumoxid, Zinkoxid oder Mischungen davon verwendet werden. Besonders bevorzugt wird Titandioxid oder Aluminiumoxid als Trägermaterial eingesetzt. Zusätzlich können diese Trägermaterialien Alkalimetalle, Erdalkalimetalle und/oder Schwefel enthalten.

Die erfindungsgemäße Kernhydrierung der Dialkylterephthalate wird vorzugsweise in mindestens einer Hydriereinheit durchgeführt. Unter einer Hydriereinheit wird in der vorliegenden Erfindung eine Einheit verstanden, die einen Reaktor oder mehrere Reaktoren, die parallel und/oder in Reihe geschaltet vorliegen können, umfassen, also einen Reaktor oder eine Reaktoranordnung, in der die Kernhydrierung stattfindet. In einer besonders bevorzugten Ausführungsform wird die Kernhydrierung in mindestens zwei hintereinandergeschalteten Hydriereinheiten durchgeführt, wobei mindestens eine der beiden Hydriereinheiten in Schlaufenfahrweise, d. h. unter Rückführung eines Teils des jeweiligen Hydrieraustrags, betrieben wird. Es kann vorteilhaft sein, wenn alle der mindestens zwei Hydriereinheiten bei der Kernhydrierung in Schlaufenfahrweise betrieben werden. Ebenso vorteilhaft kann es sein, wenn die letzte Hydriereinheit im geraden Durchgang betrieben wird.

In einer besonders bevorzugten Ausführungsform wird die Kernhydrierung in mindestens drei hintereinandergeschalteten Hydriereinheiten durchgeführt, wobei mindestens die ersten beiden Hydriereinheiten in Schlaufenfahrweise betrieben werden. Auch die letzte Hydriereinheit kann in Schlaufenweise betrieben werden, was also einer Ausführungsform entspricht, bei der alle der mindestens drei Hydriereinheiten in Schlaufenfahrweise betrieben werden. Ebenso kann die letzte Hydriereinheit im geraden Durchgang betrieben werden.

Eine weitere besonders bevorzugte Ausführungsform ist die parallele Anordnung der Reaktoren, beispielsweise in einem Rohrbündelreaktor.

Die einzelnen Reaktoren können dabei adiabatisch, polytrop oder praktisch isotherm, d. h. mit einem Temperaturanstieg (Differenz der Temperatur am Einlass und der Temperatur am Auslass des Reaktors) von typischerweise kleiner als 15 K betrieben werden. Dabei werden insbesondere die in Schlaufenfahrweise betriebenen Reaktoren bevorzugt quasi isotherm gefahren, also vorzugsweise mit einem Temperaturanstieg kleiner 15 K betrieben. Bei Reaktoren, die nicht in Schlaufenfahrweise betrieben werden, liegt der bevorzugte Temperaturanstieg im Reaktor bei unter 35 K, besonders bevorzugt unter 25 K. Zwischen einzelnen Hydrierelementen kann eine Kühlvorrichtung angebracht sein, um die Temperatur vor dem Eintritt in die folgende Hydriereinheit abzusenken.

Die erfindungsgemäße Kernhydrierung der Dialkylterephthalate wird bevorzugt in der Flüssig/Gas-Mischphase oder Flüssigphase in Dreiphasenreaktoren im Gleichstrom durchgeführt, wobei das Wasserstoff-haltige Gas in an sich bekannter Weise im flüssigen Edukt/Produktstrom verteilt wird. Im Interesse einer gleichmäßigen Flüssigkeitsverteilung, einer verbesserten Reaktionswärmeabfuhr und/oder einer hohen Raum-Zeit-Ausbeute werden die in Schlaufenfahrweise betriebenen Reaktoren vorzugsweise mit hohen Flüssigkeitsbelastungen von 10 bis 400, bevorzugt von 20 bis 200 und besonders bevorzugt von 40 bis 150 $m^3$ pro $m^2$ Querschnitt des leeren Reaktors und Stunde gefahren. Die Flüssigkeitsbelastungen können in den in Schlaufenfahrweise betriebenen Reaktoren gleich oder verschieden sein. Vorzugsweise ist die Flüssigkeitsbelastung im ersten Reaktor am größten und nimmt in den nachfolgenden in Schlaufenfahrweise betriebenen Reaktoren ab. Einer oder mehrere Reaktoren können dabei teilweise mit Flüssigkeit geflutet sein oder komplett als Rieselbettreaktoren fungieren.

Die Kernhydrierung der Dialkylterephthalate kann in Abwesenheit oder in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten. Folgende Substanzen können als Lösemittel bei der Kernhydrierung eingesetzt werden: Geradkettige oder cyclische Ether, wie beispielsweise Tetrahydrofuran oder Dioxan, sowie aliphatische Alkohole, in denen der Alkylrest 1 bis 13 Kohlenstoffatome aufweist. Bevorzugt als Lösemittel verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole. Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Eduktkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt der Eduktgehalt im Reaktorzulauf zwischen 1 und 70 %, Der gewünschte Konzentrationsbereich kann bei denjenigen Reaktoren, die in Schlaufenfahrweise betrieben werden, durch das Kreislaufverhältnis (Mengenverhältnis von rückgeführten Hydrieraustrag zu Edukt) eingestellt werden. Die Eduktkonzentrationen im Reaktorzulauf nehmen vorzugsweise vom ersten bis zum letzten Reaktor ab.

Die Kernhydrierung der Dialkylterephthalate wird erfindungsgemäß bevorzugt in einem Druckbereich von 3 bis 300 bar, insbesondere von 15 bis 200 bar, ganz besonders bevorzugt von 50 bis 200 bar durchgeführt. Der Druck kann in den einzelnen Reaktoren gleich oder verschieden sein. Vorzugsweise sind die Drücke gleich oder annähernd gleich, d. h. weichen maximal um 10% voneinander ab.

Die Hydriertemperaturen liegen bei der Kernhydrierung bevorzugt im Bereich von 50 bis 250 °C, vorzugsweise im Bereich von 80 bis 200 °C. Die Hydriertemperaturen können in einzelnen Reaktoren gleich oder verschieden sein.

Als Produkte des erfindungsgemäßen Verfahrens werden entsprechende Zusammensetzungen erhalten, die von den Einsatzstoffen und dem Umsatz bei der Hydrierung abhängig sind. Die Zusammensetzung, die bei der erfindungsgemäßen Kernhydrierung entsteht, hat vorzugsweise einen Gehalt an 1,4-Cyclohexandicarbonsäuredialkylestern von über 96 Gew.-%, insbesondere von über 98 Gew.-%, besonders bevorzugt von über 99 Gew.-%. Dieses Gemisch kann direkt oder nach Reinigung eingesetzt werden. Die Abtrennung von Nebenprodukten kann beispielsweise durch Destillation oder durch Strippen mit Wasserdampf oder mit einem Inertgas wie Stickstoff erfolgen. Bevorzugt werden geringe Mengen an Leichtsieder durch Strippen mit Wasserdampf im Temperaturbereich von 120 °C bis 240 °C, insbesondere im Bereich von 150 bis 200 °C und vorzugsweise bei einem Druck von 0,05 bis 0,1 bar abgetrennt.

Die bei der Kernhydrierung eingesetzten Dialkylterephthalate, bei denen die beiden Alkylgruppen mindestens 2 Kohlenstoffatome, vorzugsweise mindestens 4 Kohlenstoffatome aufweisen, können durch Umesterung von Dimethylterephthalat mit einem Alkohol oder Alkoholgemischen mit mehr als 2 Kohlenstoffatomen, vorzugsweise mit 3 bis 10 Kohlenstoffatomen, weiterhin bevorzugt mit 4 bis 10 Kohlenstoffatomen, weiterhin bevorzugt mit 5 bis 9 Kohlenstoffatomen, besonders bevorzugt mit 8 oder 9 Kohlenstoffatomen und ganz besonders bevorzugt mit 9 Kohlenstoffatomen hergestellt werden. Eine weitere Möglichkeit zur Herstellung der bei der Kernhydrierung eingesetzten Dialkylterephthalate ist die Veresterung von Terephthalsäure mit einem Alkohol oder einem Alkoholgemisch mit mehr als 2 Kohlenstoffatomen, vorzugsweise mit 3 bis 10 Kohlenstoffatomen, weiterhin bevorzugt mit 4 bis 10 Kohlenstoffatomen, weiterhin bevorzugt mit 5 bis 9 Kohlenstoffatomen, besonders bevorzugt mit 8 oder 9 Kohlenstoffatomen und ganz besonders bevorzugt mit 9 Kohlenstoffatomen. Beide Verfahren sind dem Fachmann grundsätzlich bekannt.

Die Umesterung von Dimethylterephthalat wird katalytisch, vorzugsweise unter Verwendung von Säuren oder Basen (nach Brönsted oder Lewis) als Katalysator, durchgeführt. Unabhängig davon welcher Katalysator eingesetzt wird, stellt sich immer ein temperaturabhängiges Gleichgewicht zwischen den Einsatzstoffen (Dimethylterephthalat und Alkohol) und den Produkten (Dialkylterephthalat und freigesetztes Methanol aus dem eingesetzten Dimethylterephthalat) ein. Um das Gleichgewicht zu Gunsten des Dialkylterephthalats zu verschieben, kann es vorteilhaft sein,das aus dem Edukt Dimethylterephthalat entstehende Methanol aus dem Reaktionsgemisch abzudestillieren.

Bei der Umesterung kann es weiterhin vorteilhaft sein, den Alkohol insgesamt im Überschuss einzusetzen. Vorzugsweise wird der eingesetzte Alkohol mit mehr als 2 Kohlenstoffatomen in einem Überschuss von 5 bis 50 %, vorzugsweise 10 bis 30 % der zur Bildung des Dialkylterephthalats notwendigen molaren Menge eingesetzt Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignete Metalle oder deren Verbindungen sind z. B. Zinn, Titan, Zirkonium, die als feinverteilte Metalle oder zweckmäßig in Form ihrer Salze, als Oxide oder in Form von löslichen organischen Verbindungen verwendet werden. Die Metallkatalysatoren sind im Vergleich zu den Katalysatoren auf Basis von Protonensäuren Hochtemperaturkatalysatoren, die ihre volle Aktivität oft erst bei Temperaturen oberhalb 180 °C erreichen. Es kann jedoch vorteilhaft sein, Metallkatalysatoren auf Basis von Metallen oder deren Verbindungen einzusetzen, um die Bildung von Nebenprodukten zu verringern oder zu vermeiden. Beispiele für besonders bevorzugt eingesetzte Metallkatalysatoren sind Zinnpulver, Zinn(II)oxid, Zinn(II)oxalat, Titansäureester wie Tetraisopropylorthotitanat oder Tetrabutylorthotitanat, sowie Zirkoniumester wie Tetrabutylzirkonat. Weiterhin können basische Katalysatoren, wie beispielsweise Oxide, Hydroxide, Hydrogencarbonate, Carbonate oder Alkoholate von Alkali- oder Erdalkalimetallen eingesetzt werden. Aus dieser Gruppe werden bevorzugt Alkoholate, wie beispielsweise Natriummethylat eingesetzt. Alkoholate können auch in situ aus einem Alkalimetall und einem Alkohol wie zum Beispiel Nonanol bzw. einem Isononanolgemisch hergestellt werden. Besonders bevorzugt werden solche Alkoholate eingesetzt, deren Alkoholrest mit einem der an der Reaktion beteiligten Alkohole übereinstimmt.

Die Katalysatorkonzentration kann in weiten Bereichen und insbesondere abhängig von der Art des Katalysators variiert werden. Die Katalysatorkonzentration beträgt vorzugsweise von 0,005 bis 2,0 Gew.-% bezogen auf das Reaktionsgemisch. Die für jeden Katalysator optimalen Konzentrationen können durch Vorversuche leicht bestimmt werden und ergeben sich aus einem Kompromiss aus möglichst geringem Katalysatorverbrauch (Kostenfaktor) und möglichst hoher Reaktionsgeschwindigkeit. Im Falle des erfindungsgemäß besonders bevorzugt eingesetzten Katalysators Titantetrabutylorthotitanates liegt die bevorzugte Konzentration beispielsweise im Bereich von 0,005 bis 1 Gew.-%, bezogen auf das eingesetzte Dimethylterephthalat.

Die Umesterung wird vorzugsweise bei einer Temperatur von 100 und 240 °C durchgeführt. Der Druck kann bei der Umesterung zwischen 0,1 und 10 bar betragen. Die Temperatur wird besonders bevorzugt so hoch gewählt, dass der aus dem Eduktester entstehende Alkohol bei dem vorgegebenen Druck aus dem Reaktionsgemisch abdestilliert werden kann.

Die hergestellten Rohestergemische können auf die gleiche Weise aufgearbeitet werden wie diejenigen, die durch die im Nachfolgenden beschriebene Veresterung von Terephthalsäure hergestellt worden sind.

Die Herstellung der Dialkylterephthalate durch Veresterung von Terephthalsäure mit einem Alkohol oder einem Alkoholgemisch mit mindestens 2 Kohlenstoffatomen, vorzugsweise mit mindestens 4 Kohlenstoffatomen kann nach allen bekannten Verfahren durchgeführt werden. Vorzugsweise erfolgt die Veresterung allerdings nach einem Verfahren, bei dem das Reaktionswasser durch azeotrope Destillation mit dem Alkohol entfernt wird und die durch die azeotrope Destillation aus der Reaktion entfernte Flüssigkeitsmenge vollständig oder teilweise mit dem Einsatzalkohol wieder ergänzt wird. Als Flüssigkeitsmenge wird im Folgenden das durch azeotrope Destillation aus der Reaktion entfernte Flüssigkeitsvolumen, hauptsächlich bestehend aus Reaktionswasser und Alkohol, bezeichnet. Ein vollständiger Ersatz der entfernten Flüssigkeitsmenge ist bevorzugt.

Die Veresterung der Terephthalsäure zu den Dialkylterephthalaten kann erfindungsgemäß autokatalysiert oder säure- bzw. basenkatalysiert durchgeführt werden. Als Veresterungskatalysatoren können Lewis- oder Brönstedsäuren oder metallorganische Stoffe, eingesetzt werden. Bevorzugte Veresterungskatalysatoren sind Alkoholate, Sulfonsäuren, Carbonsäuresalze oder Chelatverbindungen von Titan oder Zirkonium, wobei das Katalysatormolekül ein oder mehrere Metallatome enthalten kann. Insbesondere werden Tetra(isopropyl)orthotitanat und Tetra(butyl)orthotitanat eingesetzt. Die Katalysatorkonzentration hängt von der Art des Katalysators ab. Bei den bevorzugt eingesetzten Titanverbindungen beträgt diese 0,005 bis 1,0 Gew.-% bezogen auf das Reaktionsgemisch, insbesondere 0,01 bis 0,3 Gew.-%.

Die erfindungsgemäße Veresterung wird vorzugsweise in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Der Katalysator kann in reiner Form oder als Lösung, bevorzugt gelöst in einem der Einsatzstoffe, zu Beginn oder erst nach Erreichen der Reaktionstemperatur eingebracht werden. Der umzusetzende Alkohol, der als Schleppmittel dient, kann im stöchiometrischen Überschuss eingesetzt werden. Bevorzugt wird ein Überschuss von 5 bis 50 %, besonders bevorzugt 10 bis 30 % bezogen auf die eingesetzte Terephthalsäure eingesetzt.

Die Reaktionstemperaturen liegen bei Verwendung von Titankatalysatoren zwischen 120 °C und 270 °C, vorzugsweise zwischen 130 °C und 270 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem Vorratsgefäß bereitstehenden Alkohol zu ersetzen. In anderen Ausführungsformen der Veresterung wird die abgetrennte Flüssigkeit zum Alkohol, vorzugsweise zum reinen Alkohol aufgearbeitet.

Nach Beendigung der Reaktion enthält das Reaktionsgemisch, das im Wesentlichen aus dem Zielprodukt Dialkylterephthalat und überschüssigem Alkohol besteht, zusätzlich auch noch Katalysator und/oder dessen Folgeprodukte und/oder geringe Mengen an Carbonsäure. Zur Aufarbeitung dieser Rohgemische wird der überschüssige Alkohol entfernt, die sauren Verbindungen neutralisiert, der Katalysator zerstört und die dabei entstandenen festen Nebenprodukte abgetrennt. Dabei wird der größte Teil des Alkohols bei Normaldruck oder im Vakuum abdestilliert. Die letzten Spuren des Alkohols können z. B. durch Wasserdampfdestillation oder Einperlen von Stickstoff, insbesondere im Temperaturbereich von 120 bis 225 °C, entfernt werden. Die Abtrennung des Alkohols kann beispielsweise als erster oder als letzter Aufarbeitungsschritt erfolgen.

Die Neutralisation der sauren Stoffe, wie Carbonsäuren, Estercarbonsäuren oder gegebenenfalls der sauren Katalysatoren, kann durch Zugabe von basisch wirkenden Verbindungen der Alkali- und/oder Erdalkalimetalle erfolgen. Diese können in Form ihrer Carbonate, Hydrogencarbonate oder Hydroxide eingesetzt werden. Das Neutralisationsmittel kann in fester Form oder bevorzugt als Lösung, insbesondere als wässrige Lösung eingesetzt werden. Die Neutralisation kann sofort nach Beendigung der Veresterungsreaktion oder nach Abdestillation der Hauptmenge des überschüssigen Alkohols durchgeführt werden. Bevorzugt ist die Neutralisation mit Natronlauge sofort nach Beendigung der Veresterungsreaktion bei Temperaturen über 150 °C. Das mit der Lauge eingebrachte Wasser kann dann zusammen mit Alkohol abdestilliert werden.

Aufgrund der Tatsache, dass sich Terephthalsäure auch bei der Siedetemperatur in dem für die Veresterung einzusetzenden Alkohol(e) nur schwer löst, kann durch einen Überdruck von maximal 20 bar, vorzugsweise maximal 10 bar, wegen der höheren Siedetemperatur die Löslichkeit und somit die Reaktionsgeschwindigkeit weiter erhöht werden. Bei der Verwendung von Dimethylterephthalat für die Umesterung bestehen diese Probleme nicht. Ausgehend von Dimethylterephthalat kann das entsprechende Terephthalat in der Regel nach kürzeren Zeiten erhalten werden als mit Terephthalsäure als Ausgangsstoff. Daher ist die Herstellung der bei der Kernhydrierung eingesetzten Dialkylterephthalate durch Umesterung ausgehend von Dimethylterephthalat besonders bevorzugt.

Der zur Herstellung des Dialkylterephthalats bei der Umesterung oder bei der Veresterung eingesetzte Alkohol ist ein Alkohol oder ein Alkoholgemisch mit mindestens 2 Kohlenstoffatomen, vorzugsweise mindestens 4 Kohlenstoffatomen. Bevorzugt sind Alkohole mit 3 bis 10 Kohlenstoffatomen, weiterhin bevorzugt mit 4 bis 10 Kohlenstoffatomen, weiterhin bevorzugt mit 5 bis 9 Kohlenstoffatomen, besonders bevorzugt mit 8 oder 9 Kohlenstoffatomen und ganz besonders bevorzugt mit 9 Kohlenstoffatomen. Die eingesetzten Alkohole sind insbesondere primäre Alkohole, insbesondere Ethanol, Propanol, Butanol, Pentanol, Hexanol, Heptanol, Octanol, vorzugsweise 2-Ethylhexanol, Nonanol, vorzugsweise Isononanol bzw. Mischungen isomerer Nonanole, 2-Propylheptanol oder Decanol. Besonders bevorzugt ist der bei der Veresterung eingesetzte Alkohol 2-Ethylhexanol oder Isononanol.

Die erfindungsgemäß hergestellten 1,4-Cyclohexandicarbonsäuredialkylester, bei denen die beiden Alkylgruppen jeweils mindestens 2 Kohlenstoffatome, vorzugsweise mindestens 4 Kohlenstoffatome aufweisen, können vorteilhaft als Weichmacher oder Teil einer Weichmacherzusammensetzung in Kunststoffen oder Kunststoffzusammensetzungen, als Zusatz in Farben oder Lacken, in Klebstoffen oder Klebstoffkomponenten, in Dichtungsmassen oder als Lösemittel verwendet werden.

Die hergestellten 1,4-Cyclohexandicarbonsäuredialkylester können auch in Mischungen mit anderen Weichmachern, insbesondere sogenannten Schnellgelierern, als Weichmacher eingesetzt werden. Der Anteil an 1,4-Cyclohexandialkylestern in dem Gemisch mit anderen Weichmachern beträgt vorzugsweise 15 bis 95 Gew.-%, besonders bevorzugt 20 bis 90 Gew.-% und ganz besonders bevorzugt 25 bis 85 Gew.-%, wobei sich die Anteile aller vorhandenen Weichmacher zu 100 Gew.-% addieren. Die genannten Zusammensetzungen aus 1,4-Cyclohexandialkylestern und anderen Weichmachern können als Weichmacherzusammensetzung in Kunststoffen und Kunststoffzusammensetzungen, Klebstoffen, Dichtungsmassen, Lacken, Farben, Plastisolen oder Tinten verwendet werden.

Die Kunststoffzusammensetzungen, die die hergestellten 1,4-Cyclohexandicarbonsäuredialkylester enthalten können, können Polymere, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxylbuttersäure (PHB), Polyhydroxylvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten enthalten. Vorzugsweise weisen die Zusammensetzungen PVC oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf. Besonders bevorzugt ist die Verwendung von PVC.

Bevorzugt enthält die Kunststoffzusammensetzung als PVC-Typ Suspensions-Masse-, Mikrosuspensions- oder Emulsions-PVC. Bezogen auf 100 Gewichtsteile Polymer enthalten die Zusammensetzungen vorzugsweise von 5 bis 200, bevorzugt von 10 bis 150 Gewichtsteile an Weichmacher.

Die Kunststoffzusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl, Gleitmittel, Treibmittel, Kicker, Antioxidanzien, Rheologie-Additive oder Biozide.

Die Kunststoffzusammensetzungen aus den 1,4-Cyclohexandicarbonsäuredialkylestern und den vorgenannten Polymermaterialien können als Kunststoffzusammensetzungen, Klebstoffe, Dichtungsmassen, Lacke, Farben, Plastisole, Kunstleder, Fußbodenbeläge, Unterbodenschutz, Gewebebeschichtungen, Dachmembranen, Tapeten oder Tinten oder zu deren Herstellung verwendet werden.

Mit den Weichmacherzusammensetzungen hergestellte Kunststoffprodukte können beispielsweise Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Dachmembranen, Tapeten, Kabel und Drahtummantelungen sein. Bevorzugte Anwendungsgebiete aus dieser Gruppe sind Lebensmittelverpackungen, Spielzeug, Medizinalartikel, Tapeten, Dachmembranen, Gewebebeschichtungen und Fußbodenbeläge.

Die Erfindung soll nachfolgend durch Beispiele erläutert werden. Die Beispiele sind ausgewählte Ausführungsformen und stellen keine Beschränkung dar.

Beispiel 1: Kernhydrierung von Diisononylterephthalat

Zur Prüfung der Abhängigkeit der Dauer der Kernhydrierung von Diisononylterephthalat wurden verschiedene DINT-Proben in einer Kernhydrierung getestet. Die folgenden Proben wurden verwendet:

Herkunft der verschiedenen DINT-Proben:

**DINT-1**

2.318 g (16,1 mol) Isononylalkohol (INA, Evonik, Reinheit > 99 %), 1.358 g (7 mol) Dimethylterephthalat (Oxxynova, Reinheit > 99,9 %) und 2,4 g Tetra-n-Butyl-Titanat-Katalysator wurden unter Stickstoff-Atmosphäre (Stickstofffluss: 6 L/h) in einem 6 L Glaskolben mit Rührer, Tauchrohr und Raschigringkolonne mit aufgesetztem Kondensator bis 240 °C erhitzt, wobei Destillat abgenommen wurde, solange die Kopftemperatur an der Kolonne nicht über 65 °C stieg. Der Reaktionsverlauf wurde per Gaschromatograph verfolgt und die Reaktion bei einer Restkonzentration von Methylestern < 0,5 % abgestellt. Nach 3 h Reaktionslaufzeit wurde die Sumpftemperatur auf 220 °C gesenkt und zunehmend Vakuum angelegt, um den Alkoholüberschuss abzudestillieren.

Danach wurde die Kolonne durch eine Destillationsbrücke ersetzt und bei 80 °C 4 g 10%ige NaOH-Lösung und 20 mL vollentsalztes Wasser zugegeben und 15 min gerührt, um den Katalysator zu zerstören. Anschließend wurde das Reaktionsgemisch auf 180 °C erhitzt und 40 min unter vollem Vakuum getrocknet, gefolgt von 2 h Strippen mit Stickstoff bei 20 mbar. Nach der Kontrolle des Restgehalts an Alkohol per GC wurde der Ansatz auf 80 °C abgekühlt und gefiltert.

Das Reaktionsprodukt Diisononylterephthalat (DINT) wurde mit einer Reinheit von 99,96% erhalten. Die CO-Zahl wurde anhand des in der Beschreibung genannten Verfahrens ermittelt und betrug 0,03 mg KOH/g.

**DINT-2**

2.318 g (16,1 mol) Isononylalkohol (INA, Evonik, Reinheit > 99 %), 1.163 g (7 mol) Terephthalsäure (Acros Organics, Reinheit > 99 %) und 2,4 g Tetra-n-Butyl-Titanat-Katalysatorwurden unter Stickstoff-Atmosphäre (Stickstofffluss: 6 L/h) in einem 6 L Glaskolben mit Rührer, Tauchrohr, Kondensator und Wasserauskreiser bis 240 °C erhitzt. Um

EP 3 945 087 B1

die Entfernung des Wassers aus dem Reaktionssystem zu beschleunigen, wurde 140 mL Cyclohexan als Schleppmittel verwendet. Die Veresterung wurde nach 11,5 h abgestellt.

Nach dem Ende der Reaktion und dem Abkühlen auf Raumtemperatur wurde der Wasserauskreiser durch eine Destillationsbrücke ersetzt und der Restalkohol unter vollem Vakuum (ca. 1-3 mbar) bei 180 °C abdestilliert. Anschließend wurden bei 80 °C 1,9 g 10%ige NaOH-Lösung und 20 mL vollentsalztes Wasser zugegeben und 15 min gerührt, um den Katalysator zu zerstören. Anschließend wurde das Reaktionsgemisch auf 180 °C erhitzt und 40 min unter vollem Vakuum getrocknet, gefolgt von 2 h Strippen mit Stickstoff bei 20 mbar. Nach der Kontrolle des Restgehalts an Alkohol per GC wurde der Ansatz auf 80 °C abgekühlt und gefiltert.

Das Reaktionsprodukt Diisononylterephthalat (DINT) wurde mit einer Reinheit von 99,75% erhalten. Die CO-Zahl wurde anhand des in der Beschreibung genannten Verfahrens ermittelt und betrug 0,06 mg KOH/g.

**DINT-3**

In einem mit Stickstoff gefüllten großtechnischen Reaktor mit ca. 50 $m^3$ Fassungsvermögen wurden 27 t Isononanol (INA, Evonik, Reinheit > 99 %) auf ca. 145 °C vorgeheizt und anschließend 5 kg Tetra-n-Butyl-Titanat-Katalysator und 18 t Dimethylterephthalat (Oxxynova, Reinheit > 99,9 %) zugegeben. Die Reaktionsmischung wurde weiter bis auf max. 220 °C erhitzt und dabei Methanol über eine Destillationskolonne abgetrennt. Der Reaktionsverlauf wurde per Gaschromatograph verfolgt und die Reaktion bei einer Restkonzentration von Methylestern < 0,5 % abgestellt.

Sobald die Reaktion abgeschlossen war, wurde der Rücklauf auf den Kolonnenkopf gestoppt und ein Vakuum (ca. 50 - 100 mbar) auf den Reaktor gezogen. In den nächsten parallel verlaufenden Schritten wurde der Katalysator mit 25%iger NaOH-Lösung zerstört und freie Säure neutralisiert.

Das hergestellte DINT wurde bei ca. 130°C unter vollem Vakuum (ca. 50 mbar) getrocknet und anschließend gefiltert. Das Reaktionsprodukt Diisononylterephthalat (DINT) wurde mit einer Reinheit von 99,7% erhalten. Die CO-Zahl wurde anhand des in der Beschreibung genannten Verfahrens ermittelt und betrug 0,07 mg KOH/g.

**DINT-4** ist das Produkt UN499 der Firma UPC, Taiwan. Die CO-Zahl wurde anhand des in der Beschreibung genannten Verfahrens ermittelt und betrug 0,21 mg KOH/g.

**DINT-5** ist eine Mischung aus DINT-4 (60 Gew.-%) und DINT-6 (40 Gew.-%). Die CO-Zahl wurde anhand des in der Beschreibung genannten Verfahrens ermittelt und betrug 0,43 mg KOH/g.

**DINT-6** ist das Produkt Kanatol-9090 der Firma KLJ Group, Indien. Die CO-Zahl wurde anhand des in der Beschreibung genannten Verfahrens ermittelt und betrug 0,57 mg KOH/g.

Kernhydrierung der DINT-Proben

Es wurde eine Batchhydrierung von verschiedenen DINT-Proben (Diisononylterephthalat) in einem Rohrreaktor mit einem Innendurchmesser von 40 mm und einer Länge von 190 mm im Kreislaufbetrieb durchgeführt. Der Rohrreaktor wird dabei im Gleichstrom mit Flüssigphase und Gasphase im Rieselbett durchströmt. Der bei der Hydrierung verwendete Katalysator war ein Schalenkatalysator bestehend aus 1 Gew.-% Ru, welches geträgert auf Titandioxid (Aerolyst 7711) vorliegt. In den Rohrreaktor wurden jeweils 25 g Hydrierkatalysator sowie 25 g Inertmaterial, bestehend aus $Al_2O_3$, in Form von 1,5 mm Extrudaten eingesetzt. Die bei der Hydrierung eingesetzte Menge an DINT betrug stets 1000 g. Die $H_2$-Regelung erfolgt über eine konstante Abgasfahrweise, wobei ein konstanter Abgasstrom von 1 L/h (47,8 $m^3m^{-2}h^{-1}$) eingestellt wurde. Alle Versuche wurden bei einem Anlagendruck von 90 bar sowie einer Rohrreaktortemperatur von 110 °C durchgeführt. Nach Passieren eines Wärmetauschers unterhalb des Reaktors erfolgt eine Gas-Flüssigtrennung mittels Abscheider. Die Gasphase wird kontinuierlich ins Abgas entspannt. Die Flüssigphase wird über einen beheizten Vorwärmer zum Rohrreaktor zurückgeführt, wo erneut eine Hydrierung mittels $H_2$ erfolgen kann. Die Konzentration von DINT wurde mittels inline-Raman-Analytik über die Zeit erfasst.

Die Ergebnisse sind der nachfolgenden Tabelle 1 zu entnehmen.

Tabelle 1: Konzentration von DINT in Abhängigkeit von der Zeit bei der Hydrierung

| Probe | CO-Zahl | c (DINT)* 0h | c (DINT) 2h | c (DINT) 4h | c (DINT) 8h | c (DINT) 12h |
|---|---|---|---|---|---|---|
| DINT-1 | 0,03 | 100 | 66,3 | 40,5 | 10,6 | 1,6 |
| DINT-2 | 0,06 | 100 | 71,1 | 47,3 | 16,3 | 3,7 |
| DINT-3 | 0,07 | 100 | 71,9 | 49,1 | 17,0 | 3,5 |
| DINT-4 | 0,21 | 100 | 76,3 | 57,1 | 27,3 | 9,6 |
| DINT-5 | 0,43 | 100 | 80,3 | 64,0 | 36,5 | 17,2 |

(fortgesetzt)

| Probe | CO-Zahl | c (DINT)* 0h | c (DINT) 2h | c (DINT) 4h | c (DINT) 8h | c (DINT) 12h |
|-------|---------|--------------|-------------|-------------|-------------|--------------|
| DINT-6 | 0,57 | 100 | 87,9 | 75,9 | 53,7 | 34,9 |
| * die Konzentration c (DINT) ist auf 100% normiert. | | | | | | |

Aus der Tabelle ist zu entnehmen, dass bei Einsatz von DINT mit einer CO-Zahl innerhalb des beanspruchten Bereichs eine deutlich schnellere Kernhydrierung erfolgt.

**Patentansprüche**

1.  Verfahren zur Herstellung von 1,4-Cyclohexandicarbonsäuredialkylestern, bei denen die beiden Alkylgruppen jeweils mindestens 2 Kohlenstoffatome, vorzugsweise mindestens 4 Kohlenstoffatome aufweisen, wobei das Verfahren mindestens das

    Kernhydrieren eines Dialkylterephthalats, bei dem die beiden Alkylgruppen jeweils mindestens 2 Kohlenstoffatome aufweisen, in Anwesenheit eines heterogenen Hydrierkatalysators mit einem Wasserstoff-haltigen Gas zum entsprechenden 1,4-Cyclohexandicarbonsäuredialkylester umfasst, **dadurch gekennzeichnet, dass** das bei der Kernhydrierung eingesetzte Dialkylterephthalat eine CO-Zahl von weniger als 0,3 mg KOH/g, vorzugsweise weniger als 0,2 mg KOH/g, besonders bevorzugt weniger als 0,1 mg KOH/g aufweist.

2.  Verfahren nach Anspruch 1, wobei die beiden Alkylgruppen des 1,4-Cyclohexandicarbonsäuredialkylesters 3 bis 10 Kohlenstoffatome, vorzugsweise 4 bis 10 Kohlenstoffatome, weiterhin bevorzugt 5 bis 9 Kohlenstoffatome, besonders bevorzugt 8 oder 9 Kohlenstoffatome, ganz besonders bevorzugt 9 Kohlenstoffatome aufweisen.

3.  Verfahren nach Anspruch 1 oder 2, wobei das bei der Kernhydrierung eingesetzte Dialkylterephthalat durch Umesterung von Dimethylterephthalat mit einem Alkohol mit mindestens 2 Kohlenstoffatomen oder durch Veresterung von Terephthalsäure mit einem Alkohol mit mindestens 2 Kohlenstoffatomen hergestellt wird.

4.  Verfahren nach Anspruch 3, wobei der bei der Umesterung oder bei der Veresterung eingesetzte Alkohol ein Alkohol mit 3 bis 10 Kohlenstoffatomen, vorzugsweise 4 bis 10 Kohlenstoffatomen, weiterhin bevorzugt mit 5 bis 9 Kohlenstoffatomen, besonders bevorzugt mit 8 oder 9 Kohlenstoffatomen, ganz besonders bevorzugt mit 9 Kohlenstoffatomen ist.

5.  Verfahren nach Anspruch 2 oder 4, wobei der 1,4-Cyclohexandicarbonsäuredialkylester ein 1,4-Cyclohexandicarbonsäurediisononylester oder ein 1,4-Cyclohexandicarbonsäuredi-2-ethylhexylester ist.

6.  Verfahren nach einem der Ansprüche 1 bis 5, wobei der bei der Kernhydrierung eingesetzte heterogene Hydrierkatalysator ein Übergangsmetall auf einem Trägermaterial umfasst.

7.  Verfahren nach Anspruch 6, wobei das Übergangsmetall ein Metall der Gruppe 8 des Periodensystems der Elemente (Eisengruppe), vorzugsweise Ruthenium ist.

8.  Verfahren nach Anspruch 6, wobei das Trägermaterial aus der Gruppe, bestehend aus Aktivkohle, Siliciumcarbid, Aluminiumoxid, Siliciumoxid, Alumosilikat, Titandioxid, Zirkoniumdioxid, Magnesiumoxid, Zinkoxid oder Mischungen davon, ausgewählt wird.

9.  Verfahren nach Anspruch 8, wobei das Trägermaterial Titandioxid oder Aluminiumoxid ist.

10. Verfahren nach einem der Ansprüche 6 bis 9, wobei der Gehalt an Übergangsmetall im heterogenen Hydrierkatalysator im Bereich von 0,1 bis 10 Gew.-%, vorzugsweise insbesondere im Bereich von 0,5 bis 5 Gew.-%, besonders im Bereich von 1 und 3 Gew.-% liegt.

11. Verfahren nach einem der Ansprüche 1 bis 10, wobei die Kernhydrierung in mindestens einer Hydriereinheit, vorzugsweise in mindestens zwei hintereinandergeschalteten Hydriereinheiten durchgeführt wird, wobei mindestens eine der mindestens zwei Hydriereinheiten in Schlaufenfahrweise betrieben wird.

12. Verfahren nach einem der Ansprüche 1 bis 11, wobei die Hydriertemperatur bei der Kernhydrierung im Bereich von 50 bis 250 °C liegt.

13. Verfahren nach einem der Ansprüche 1 bis 12, wobei die Kernhydrierung in einem Druckbereich von 3 bis 300 bar durchgeführt wird.

**Claims**

1. Process for preparing dialkyl 1,4-cyclohexanedicarboxylates in which the two alkyl groups both have at least 2 carbon atoms, preferably at least 4 carbon atoms, the process comprising at least the

   ring hydrogenation of a dialkyl terephthalate in which the two alkyl groups both have at least 2 carbon atoms, in the presence of a heterogeneous hydrogenation catalyst, with a hydrogen-containing gas to form the corresponding dialkyl 1,4-cyclohexanedicarboxylate, **characterized in that**
   the dialkyl terephthalate used in the ring hydrogenation has a CO value of less than 0.3 mg KOH/g, preferably less than 0.2 mg KOH/g, more preferably less than 0.1 mg KOH/g.

2. Process according to Claim 1, wherein the two alkyl groups of the dialkyl 1,4-cyclohexanedicarboxylate have 3 to 10 carbon atoms, preferably 4 to 10 carbon atoms, more preferably 5 to 9 carbon atoms, particularly preferably 8 or 9 carbon atoms and most preferably 9 carbon atoms.

3. Process according to Claim 1 or 2, wherein the dialkyl terephthalate used in the ring hydrogenation is prepared by transesterification of dimethyl terephthalate with an alcohol having at least 2 carbon atoms or by esterification of terephthalic acid with an alcohol having at least 2 carbon atoms.

4. Process according to Claim 3, wherein the alcohol used in the transesterification or in the esterification is an alcohol having 3 to 10 carbon atoms, preferably 4 to 10 carbon atoms, more preferably having 5 to 9 carbon atoms, particularly preferably having 8 or 9 carbon atoms and most preferably having 9 carbon atoms.

5. Process according to Claim 2 or 4, wherein the dialkyl 1,4-cyclohexanedicarboxylate is a diisononyl 1,4-cyclohexanedicarboxylate or a di-2-ethylhexyl 1,4-cyclohexanedicarboxylate.

6. Process according to any of Claims 1 to 5, wherein the heterogeneous hydrogenation catalyst used in the ring hydrogenation comprises a transition metal on a support material.

7. Process according to Claim 6, wherein the transition metal is a metal of group 8 of the periodic table of the elements (iron group), preferably ruthenium.

8. Process according to Claim 6, wherein the support material is selected from the group consisting of activated carbon, silicon carbide, aluminium oxide, silicon oxide, aluminosilicate, titanium dioxide, zirconium dioxide, magnesium oxide, zinc oxide or mixtures thereof.

9. Process according to Claim 8, wherein the support material is titanium dioxide or aluminium oxide.

10. Process according to any of Claims 6 to 9, wherein the transition metal content in the heterogeneous hydrogenation catalyst is within a range from 0.1% to 10% by weight, preferably in particular within a range from 0.5% to 5% by weight, particularly within a range from 1% to 3% by weight.

11. Process according to any of Claims 1 to 10, wherein the ring hydrogenation is carried out in at least one hydrogenation unit, preferably in at least two hydrogenation units connected in series, wherein at least one of the at least two hydrogenation units is operated in loop mode.

12. Process according to any of Claims 1 to 11, wherein the hydrogenation temperature in the ring hydrogenation is within a range from 50 to 250°C.

**EP 3 945 087 B1**

**13.** Process according to any of Claims 1 to 12, wherein the ring hydrogenation is carried out within a pressure range from 3 to 300 bar.

**Revendications**

**1.** Procédé pour la préparation d'esters dialkyliques d'acide 1,4-cyclohexanedicarboxylique, dans lesquels les deux groupes alkyle comportent chacun au moins 2 atomes de carbone, de préférence au moins 4 atomes de carbone, le procédé comprenant au moins

l'hydrogénation au noyau d'un téréphtalate de dialkyle, dans lequel les deux groupes alkyle comportent chacun au moins 2 atomes de carbone, en présence d'un catalyseur d'hydrogénation hétérogène, avec un gaz contenant de l'hydrogène, conduisant à l'ester dialkylique d'acide 1,4-cyclohexanedicarboxylique correspondant, **caractérisé en ce que**
le téréphtalate de dialkyle utilisé dans l'hydrogénation au noyau présente un indice de CO de moins de 0,3 mg de KOH/g, de préférence moins de 0,2 mg de KOH/g, de façon particulièrement préférée moins de 0,1 mg de KOH/g.

**2.** Procédé selon la revendication 1, dans lequel les deux groupes alkyle de l'ester dialkylique d'acide 1,4-cyclohexanedicarboxylique comportent de 3 à 10 atomes de carbone, de préférence 4 à 10 atomes de carbone, encore mieux 5 à 9 atomes de carbone, de façon particulièrement préférée 8 ou 9 atomes de carbone, de façon tout particulièrement préférée 9 atomes de carbone.

**3.** Procédé selon la revendication 1 ou 2, dans lequel le téréphtalate de dialkyle utilisé dans l'hydrogénation au noyau est préparé par transestérification de téréphtalate de diméthyle par un alcool ayant au moins 2 atomes de carbone ou par estérification d'acide téréphtalique par un alcool ayant au moins 2 atomes de carbone.

**4.** Procédé selon la revendication 3, dans lequel l'alcool utilisé dans la transestérification ou dans l'estérification est un alcool ayant de 3 à 10 atomes de carbone, de préférence 4 à 10 atomes de carbone, encore mieux ayant de 5 à 9 atomes de carbone, de façon particulièrement préférée ayant 8 ou 9 atomes de carbone, de façon tout particulièrement préférée ayant 9 atomes de carbone.

**5.** Procédé selon la revendication 2 ou 4, dans lequel l'ester dialkylique d'acide 1,4-cyclohexanedicarboxylique est un ester diisononylique d'acide 1,4-cyclohexanedicarboxylique ou un ester di-2-éthylhexylique d'acide 1,4-cyclohexanedicarboxylique.

**6.** Procédé selon l'une quelconque des revendications 1 à 5, dans lequel le catalyseur d'hydrogénation hétérogène utilisé dans l'hydrogénation au noyau comprend un métal de transition sur une matière de support.

**7.** Procédé selon la revendication 6, dans lequel le métal de transition est un métal du groupe 8 du système périodique des éléments (groupe du fer), de préférence le ruthénium.

**8.** Procédé selon la revendication 6, dans lequel la matière de support est choisie dans le groupe constitué par le charbon actif, le carbure de silicium, l'oxyde d'aluminium, l'oxyde de silicium, un aluminosilicate, le dioxyde de titane, le dioxyde de zirconium, l'oxyde de magnésium, l'oxyde de zinc ou des mélanges de ceuxci .

**9.** Procédé selon la revendication 8, dans lequel la matière de support est le dioxyde de titane ou l'oxyde d'aluminium.

**10.** Procédé selon l'une quelconque des revendications 6 à 9, dans lequel la teneur en métal de transition du catalyseur d'hydrogénation hétérogène se situe dans la plage de 0,1 à 10 % en poids, de façon particulièrement préférée dans la plage de 0,5 à 5 % en poids, en particulier dans la plage de 1 à 3 % en poids.

**11.** Procédé selon l'une quelconque des revendications 1 à 10, dans lequel l'hydrogénation au noyau est effectuée dans au moins une unité d'hydrogénation, de préférence dans au moins deux unités d'hydrogénation raccordées en série, au moins une desdites au moins deux unités d'hydrogénation étant utilisée en mode de circulation en boucle.

**12.** Procédé selon l'une quelconque des revendications 1 à 11, dans lequel la température d'hydrogénation dans l'hydrogénation au noyau se situe dans la plage de 50 à 250 °C.

**13.** Procédé selon l'une quelconque des revendications 1 à 12, dans lequel l'hydrogénation au noyau est effectuée dans une plage de pression de 3 à 300 bars.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3085686 A